# EUROPÄISCHE PATENTSCHRIFT

(11) **EP 3 448 323 B1**
(45) Veröffentlichungstag und Bekanntmachung des Hinweises auf die Patenterteilung: **03.06.2026**
(21) Anmeldenummer: 17719845.4
(22) Anmeldetag: 28.04.2017
(51) Int. Cl.: A61F 2/78, A61F 2/80

(54) **PROTHESENSCHAFTSYSTEM SOWIE PROTHESENSCHAFT UND LINER**
PROSTHETIC SOCKET SYSTEM, AND PROSTHETIC SOCKET AND LINER
SYSTÈME D'EMBOÎTURE DE PROTHÈSE, EMBOÎTURE DE PROTHÈSE ET MANCHON

(30) Priorität: 29.04.2016 DE 102016108046
(43) Veröffentlichungstag der Anmeldung: 06.03.2019
(73) Patentinhaber: Otto Bock Healthcare Products GmbH, 1070 Wien (AT)
(72) Erfinder: PAWLIK, Roland, 1130 Wien (AT); NIEUWENDIJK, Johan, 1140 Wien (AT); GLORIEUX, Dries, 3920 Lommel (BE); MOSLER, Lüder, 37115 Duderstadt (DE)
(74) Vertreter: Gramm, Lins & Partner Patent- und Rechtsanwälte PartGmbB
(86) Internationale Anmeldenummer: PCT/EP2017/060174
(87) Internationale Veröffentlichungsnummer: WO 2017/186902

(56) Entgegenhaltungen:
- DE-A1- 102011 116 280
- DE-A1- 102014 001 000
- US-A1- 2010 004 756
- US-A1- 2014 277 584
- US-A1- 2015 105 867

## Beschreibung

Die Erfindung betrifft ein Prothesenschaftsystem mit einem Prothesenschaft und einem Liner, wobei der Prothesenschaft einen distalen Abschlussbereich, zumindest eine sich davon in Proximalrichtung erstreckende Schaftwand und Anschlussmittel zur Befestigung einer Prothesenkomponente aufweist, wobei die Schaftwand einen in dem Prothesenschaft aufgenommenen Stumpf im angelegten Zustand zumindest teilweise umschließt und der Liner eine proximale Einstiegsöffnung und eine sich in distale Richtung erstreckende Seitenwand aufweist, die einen Stumpf im angelegten Zustand zumindest teilweise umgibt und im angelegten Zustand zwischen dem Stumpf und dem Prothesenschaft angeordnet ist und zwischen dem Liner und dem Prothesenschaft in dem distalen Abschlussbereich eine Kopplungseinrichtung angeordnet ist, wobei an der Außenseite der Seitenwand des Liners und an der Innenseite der Schaftwand miteinander in Wechselwirkung tretende Widerstandsbereiche angeordnet oder ausgebildet sind, die einer Auszugsbewegung des Liners in Proximalrichtung aus dem Prothesenschaft entgegenwirken.

Prothesenschäfte dienen zur Festlegung einer Prothesenkomponente, beispielsweise einer Gelenkeinrichtung, eines Prothesenfußes oder einer Prothesenhand, an einem Stumpf einer Gliedmaße. Der Prothesenschaft stellt dazu eine ausreichend formstabile Schaftwand bereit, die den Stumpf umfänglich zumindest teilweise umschließt. Über die Schaftwand und einen distalen Abschlussbereich werden die Kräfte von der Prothesenkomponente auf den Stumpf übertragen. Um den Tragekomfort zu erhöhen und um den Prothesenschaft an dem Stumpf festlegen zu können, ist ein Liner oder Prothesenliner vorgesehen, der an dem Stumpf anliegt. Der Liner besteht in der Regel aus einem elastischen Material und weist eine proximale Einstiegsöffnung auf. Als Linermaterial wird häufig Silikon oder ein anderer Polymer eingesetzt, das an der Hautoberfläche haftet. Eine Kopplung zwischen dem Prothesenschaft und dem Liner kann über ein distal angeordnetes Verriegelungssystem, ein sogenanntes Pin-Lock oder über ein Unterdrucksystem erfolgen.

Aus der US 8,999,004 B2 ist eine magnetische Kopplungsvorrichtung einer Prothese bekannt, mit einem Schaft und einem Liner. Innerhalb des Schaftes ist ein Permanentmagnet angeordnet. An dem Liner ist eine Montageplatte angeordnet, die durch den Permanentmagneten je nach Orientierung in den Prothesenschaft hineingezogen oder daraus herausgedrückt wird.

Die DE 10 2011 116 280 A1 betrifft ein Verbindungssystem zum lösbaren Verbinden eines Prothesenschaftes mit einem über einen Amputationsstumpf gezogenen Liner mit einem Verbindungsstift und einer Aufnahmeeinrichtung, in die der Verbindungsstift einführbar ist. Der Verbindungsstift umfasst wenigstens zwei starre Stiftsegmente, die entlang einer Längsrichtung des Verbindungsstiftes gegeneinander verlagerbar hintereinander angeordnet sind.

Die US 2015/0105867 A1 betrifft ein Prothesensystem mit einem Prothesenschaft und mit einem Prothesenliner, wobei auf der Außenseite des Prothesenliners und auf der Innenseite des Prothesenschaftes komplementäre Formschlusselemente angeordnet oder ausgebildet sind.

Die DE 10 2014 001 000 A1 betrifft eine Verbindungseinrichtung zum Verbinden zumindest einer prothetischen Einrichtung mit einem Körperstumpf einer Person. Die Verbindungseinrichtung ist eine bewegungshemmende Einrichtung in zumindest einer Richtung bezüglich einer Relativbewegung zwischen der prothetischen Einrichtung und dem Körperstumpf ausgebildet. Sie ermöglicht eine feste Verbindung der prothetischen Einrichtung und des Körperstumpfes und verhindert ein Trennen. Die Verbindungseinrichtung kann schuppenartig ausgebildet sein.

Die US 2010/0004756 A 1 betrifft einen Liner mit einer Innenseite und einer Außenseite, an denen Fasern angeordnet sind, um den Liner an einem Stumpf und/oder der Innenseite eines Protheseschaftes, an der ebenfalls Fasern angeordnet sind, festzulegen. Die Fasern ermöglichen ein Einführen in den Prothesenschaft und stellen einen vergrößerten Widerstand gegen eine Auszugsbewegung bereit.

Aufgabe der vorliegenden Erfindung ist es, ein Prothesenschaftsystem, einen Prothesenschaft sowie einen Liner bereitzustellen, mit denen ein verbesserter Tragkomfort und ein erleichtertes Einsteigen in den Prothesenschaft möglich sind.

Erfindungsgemäß wird diese Aufgabe durch ein Prothesenschaftsystem mit den Merkmalen des Hauptanspruches sowie ein Prothesenschaft und ein Liner mit den Merkmalen der nebengeordneten Ansprüche gelöst. Vorteilhafte Ausgestaltungen und Weiterbildungen in der Erfindung sind in den Unteransprüchen, der Beschreibung sowie den Figuren offenbart.

Das erfindungsgemäße Prothesenschaftsystem mit einem Prothesenschaft und mit einem Liner, wobei der Prothesenschaft einem distalen Abschlussbereich, zumindest einer sich davon in Proximalrichtung erstreckende Schaftwand und Anschlussmittel zur Befestigung einer Prothesenkomponente aufweist, wobei die Schaftwand einen in dem Prothesenschaft aufgenommenen Stumpf im angelegten Zustand zumindest teilweise umschließt und der Liner in eine proximale Einstiegsöffnung und eine sich in distaler Richtung erstreckende Seitenwand aufweist, die einen Stumpf im angelegten Zustand zumindest teilweise umgibt und im angelegten Zustand zwischen dem Stumpf und dem Prothesenschaft angeordnet ist, wobei zwischen dem Liner und dem Prothesenschaft in dem distalen Abschlussbereich eine Kopplungseinrichtung angeordnet ist, sieht vor, dass an der Außenseite der Seitenwand des Liners und an der Innenseite der Schaftwand miteinander in Wechselwirkung tretende Widerstandsbereiche angeordnet oder ausgebildet sind, die einer Auszugsbewegung des Liners in Proximalrichtung aus dem Prothesenschaft entgegenwirken. Die Widerstandsbereiche sind mit gleichgerichteten knickstabilen Noppen, Stegen oder Fasern ausgestattet. Durch die Kopplungseinrichtung im distalen Abschlussbereich des Prothesenschaftes wird eine form- oder kraftschlüssige Kopplung des distalen Abschlussbereiches des Prothesenschaftes mit dem Liner, bevorzugt mit einer distalen Abschlusskappe des Liners, bereitgestellt, mit der verhindert wird, dass während der Schwungphase oder während einer Zugbelastung des Prothesenschaftes von dem Stumpf weg der Liner sich dort von dem Prothesenschaft löst. Durch die Kopplungseinrichtung wird ein Kontaktverlust im distalen Bereich verhindert. Eine solche Kopplung zwischen dem Liner
und dem Prothesenschaft ist insbesondere bei Prothesen der unteren Extremität vorteilhaft, da dort vergleichsweise große Massen und große Beschleunigungen auftreten, sodass beispielsweise während der Schwungphase große Zugkräfte auf das Prothesenschaftsystem ausgeübt werden. Zusätzlich zu einer distalen Kopplungseinrichtung ist an dem Außenumfang des Liners und korrespondierend dazu an dem Innenumfang der Schaftwand jeweils zumindest ein Widerstandsbereich angeordnet oder ausgebildet, der in Wechselwirkung mit dem korrespondierenden Widerstandsbereich einer Auszugsbewegung des Liners aus dem Prothesenschaft entgegenwirkt. Zusätzlich zu der Haltekraft durch die Kopplungseinrichtung wird über die Seitenwand und die Schaftwand über die Umfangsfläche verteilt großflächig eine Kraftübertragung von dem Prothesenschaft über den Liner auf den Stumpf bereitgestellt, sodass eine effektive Festlegung des Prothesenschaftes an der Gliedmaße erfolgen kann.

Um nach einem Anlegen des Prothesenschaftes an dem Stumpf diesen wieder ablegen zu können, ist der Prothesenschaft bevorzugt aufweitbar ausgestaltet, wodurch es möglich ist, den Schaftumfang zu vergrößern, die Wechselwirkung der Widerstandsbereiche aufzuheben und dadurch ein Aussteigen aus dem Prothesenschaft zu erleichtern.

Eine Weiterbildung der Erfindung sieht vor, dass der Prothesenschaft mehrere, zu einander verschwenkbare oder klappbare Schaftwandkomponenten aufweist, die beispielsweise an einem Träger oder einer formstabilen Endstruktur angeordnet sind. Die Endstruktur kann schüsselartig oder kappenartig ausgebildet sein und klappbare Befestigungselemente für die Schaftwandkomponenten aufweisen. Zumindest eine Schaftwandkomponente kann klappbar an dem Träger oder der Endstruktur angeordnet sein, beispielsweise über ein Scharniergelenk, um so eine Umfangsvergrößerung zu ermöglichen. Dadurch werden die Widerstandsbereiche außer Eingriff miteinander gebracht oder die Wechselwirkung der Widerstandsbereiche aufgehoben, sodass nach dem Öffnen des Prothesenschaftes nur noch die Magnetkopplung aufgelöst werden muss, um den Liner aus dem Prothesenschaft zu entfernen.

Eine Weiterbildung der Erfindung sieht vor, dass die Kopplungseinrichtung an dem distalen Abschlussbereich oder Endbereich als Magnetkopplung ausgebildet ist. Durch die Magnetkopplung im distalen Abschlussbereich des Prothesenschaftes wird eine rein kraftschlüssige Kopplung des distalen Abschlussbereiches des Prothesenschaftes mit dem Liner, bevorzugt mit einer distalen Abschlusskappe des Liners, bereitgestellt. Durch die Magnetkopplung wird ein als unangenehm empfundener Kontaktverlust im distalen Bereich verhindert. Über den oder die Magneten kann eine einfache, kraftschlüssige und großflächige Festlegung des Liners an den Prothesenschaft erfolgen. Es müssen keine mechanischen Verriegelungseinrichtungen aktiviert oder deaktiviert werden. Die Ausrichtung des Liners relativ zu dem Prothesenschaft ist fehlerverzeihend und darüber hinaus wird die Einstiegsbewegung oder Einzugsbewegung in den Prothesenschaft unterstützt. Die Kopplungseinrichtung kann auch als mechanische Verriegelung ausgebildet sein, beispielsweise als Pinlock oder als Klettverschluss oder eine andere, formschlüssige Verriegelung, bei der sich miteinander verhakende Elemente aneinander festlegen. Die Verriegelung über Klettverschluss oder ähnliche Systeme hat den Vorteil gegenüber einem einzelnen Verriegelungselement, dass die Zuordnung bei dem Einführen nicht so präzise erfolgen muss und darüber hinaus eine Drehsicherung bereitgestellt wird.

An dem Prothesenschaft kann zumindest eine Spanneinrichtung angeordnet sein, die eine Umfangsverringerung der Schaftwand bewirkt. Über die Spanneinrichtung können beispielsweise Schaftwandkomponenten aufeinander zu verlagert werden, sodass einerseits eine Anpassung an unterschiedliche Stumpfvolumina und andererseits die Widerstandsbereiche in Wechselwirkung oder im Eingriff miteinander gebracht werden können. Die Spanneinrichtung kann als Gurtsystem oder als Kabelsystem mit einer Aufwickel- oder Abwickeleinrichtung ausgebildet sein, um beispielsweise durch Drehen der Spanneinrichtung die wirksame Länge des Gurtes oder des Kabels zu vergrößern oder zu verlängern. Dadurch werden beispielsweise Schaftwandkomponenten aufeinander zu bewegt oder voneinander weg verlagert. Neben der Ausgestaltung mit mehreren Schaftwandkomponenten sieht eine Ausgestaltungsform der Erfindung vor, dass die Schaftwand an einer Stelle einen offenen Querschnitt aufweist und sich die einander gegenüberliegenden Ränder der Schaftwand überlappen, sodass durch Aktivieren der Spanneinrichtung der Umfang des Prothesenschaftes verringert oder vergrößert wird.

Der Prothesenschaft kann in oder entgegen einer radialen Aufweitbewegung elastisch vorgespannt sein, sodass der Prothesenschaft entgegen einer Vorspannkraft über die Spanneinrichtung in seinem Umfang verringert werden muss. Dadurch wird das Öffnen des Prothesenschaftes erleichtert. Alternativ kann eine Vorspannkraft in Richtung auf eine Umfangsverringerung aufgebracht sein, sodass zum Einsteigen der Prothesenschaft gegen eine Vorspannkraft geöffnet werden muss, sodass sich der Prothesenschaft nach dem Loslassen selbstständig an den Liner anlegt.

Der Prothesenschaft kann selbstschließend ausgebildet sein, beispielsweise durch ein Gurtsystem, das an einer klappbaren Schaftwandkomponente angeordnet ist und bei Einführen des Liners in den Prothesenschaft eine Verschwenkung der Komponenten aufeinander zu bewirkt. Durch eine entsprechende Gurtführung kann auch eine in Umfangsrichtung wirkende Spann- oder Schließkraft bei einem Einstieg in den Prothesenschaft auf die Schaftwand aufgebracht werden.

Der Prothesenschaft kann modular mit einem Träger und daran befestigen, separat gefertigten Schaftwandkomponenten ausgebildet sein. Die Schaftwandkomponenten können an dem Träger reversibel oder irreversibel festgelegt sein. Über die modulare Ausgestaltung ist es möglich, industriell vorgefertigte Teile zu verwenden und diese insbesondere hinsichtlich der Prothesenschaftlänge einstellbar auszugestalten. Durch Kürzen der Komponenten ist eine Längenanpassung der Gesamtschaftlänge einfach möglich.

**In** dem distalen Abschlussbereich und/oder in einem distalen Linerendbereich können Magnete in alternierender Polung angeordnet sein, um eine Ausrichtung und Orientierung des Prothesenschaftes relativ zu dem Liner auch in Umfangsrichtung bereitzustellen. Durch die entsprechende Polung der Magnete ergibt sich eine feste Zuordnung des Prothesenschaftes zu dem Liner, sodass auf eine zusätzliche Verdrehsicherung verzichtet werden kann.

Die Widerstandsbereiche können mit Formschlusselementen und/oder Adhäsionsbereichen ausgebildet oder ausgestattet sein. Die Formschlusselemente können als Steigfell, Strichvelour oder auch Klettverschlüsse ausgebildet sein. Die Widerstandsbereiche können als oder mit Noppen, Rillen, Vorsprünge, Hinterschneidungen Verhakungsbereiche, Aufrauhungen oder andere Formgebungen ausgebildet sein. Zusätzlich oder alternativ können Widerstandsbereiche als Adhäsionsbereiche ausgebildet sein, die ein Anhaften des Liners an dem Prothesenschaft bewirken. Durch das Aufklappen und/oder die Umfangserweiterung ist es möglich, die jeweiligen Widerstandsbereiche außer Wechselwirkung oder außer Eingriff miteinander zu bringen, sodass auch ein Aussteigen aus dem Prothesenschaft ermöglicht wird.

In dem distalen Abschlussbereich können Permanentmagnete oder zumindest ein ferromagnetisches Element angeordnet sein. In dem Linerendbereich ist dann die jeweils korrespondierende Komponente zur Bildung einer Magnetkopplung mit dem Prothesenschaft angeordnet, also zumindest ein ferromagnetisches Element bei einem Permanentmagnet oder aber ein Permanentmagnet, wenn in dem Abschlussbereich ein ferromagnetisches Element angeordnet ist. Bei der Anordnung von Permanentmagneten sowohl im Abschlussbereich als auch im Linerendbereich ist auf eine entsprechende Polorientierung zu achten, um eine sichere Magnetkopplung bereitzustellen.

In dem distalen Abschlussbereich des Prothesenschaftes kann eine Metallplatte mit einer Befestigungseinrichtung, beispielsweise einem Gewinde, zur Festlegung der distalen Anschlussmittel angeordnet sein. Die Anschlussmittel sind insbesondere ein Pyramidenadapter oder eine Aufnahme für den Pyramidenadapter.

Eine Weiterbildung der Erfindung sieht vor, dass die Widerstandsbereiche schaltbar ausgebildet sind und/oder eine Trenneinrichtung zwischen dem Prothesenschaft und dem Prothesenliner positionierbar, insbesondere einschiebbar ist, so dass eine Trennschicht oder ein Trennelement zumindest bereichsweise zwischen die Widerstandsbereiche bringbar ist. Die Widerstandsbereiche oder zumindest ein Widerstandsbereich kann Elemente aufweisen, die in Einführrichtung in den Prothesenschaft geneigt sind oder aus einer Verriegelungsstellung in eine Freigabestellung oder umgekehrt gebracht werden können. Dazu können die Elemente in eine andere Orientierung oder in eine exponierte und zurückgezogene Stellung verlagert werden. Dadurch wird erreicht, dass die Widerstandskraft gegen die Auszugsbewegung verringert und ein Ausziehen oder Aussteigen aus dem Prothesenschaft erleichtert wird. Die Verlagerung oder Umorientierung der Elemente in den Widerstandsbereichen kann mechanisch oder auf eine andere Art und Weise erfolgen, beispielsweise über schaltbare Magnete. Die Trenneinrichtung ist beispielsweise als Schieber ausgebildet, der an dem Prothesenschaft verlagerbar festgelegt oder aber als separates Bauteil ausgebildet ist. Die Trenneinrichtung kann an dem Prothesenschaft verschieblich oder drehbar gelagert sein, beispielsweise in einer Tasche oder einem Aufnahmeraum, der neben dem Widerstandbereich angeordnet ist. Soll dann eine Trennung der Widerstandsbereiche erfolgen, wird die Trenneinrichtung zwischen die Widerstandsbereiche geschoben, so dass nur noch die Magnetkräfte überwunden werden müssen, um aus dem Prothesenschaft auszusteigen. Die Ausgestaltung des Prothesenschaftsystems mit Prothesenschaft und Prothesenliner mit miteinander wechselwirkenden Widerstandsbereichen und einer Trenneinrichtung kann auch unabhängig von einer zusätzlichen Magnetkopplung eingesetzt werden und ist als eigenständige Lösung ausgebildet.

Die Widerstandsbereiche können gelenkig gelagerte oder flexible Sperrkörper oder Widerstandselemente aufweisen, die aufgrund ihrer Ausrichtung oder Position ein progressives Reibsystem mit der Außenseite des Prothesenliners bilden. Zum Aussteigen aus dem Prothesenschaft können diese Sperrkörper oder Widerstandselemente, die beispielsweise als Stege, Borsten, Schuppen, Stifte oder Vorsprünge ausgebildet sein können, entriegelt und außer Eingriff, Kontakt oder Wechselwirkung mit dem Liner gebracht werden.

Die Sperrkörper oder Widerstandselemente können durch die Trenneinrichtung hindurchragen oder in der Widerstandsstellung über die Trenneinrichtung hinausragen. Dazu sind beispielsweise Ausnehmungen in der Trenneinrichtung vorhanden, durch die Sperrkörper oder Widerstandselemente ragen. Zum Verringern oder Auflösen der Widerstandes oder zur Trennung der Widerstandsbereiche voneinander wird die Trenneinrichtung in eine Entriegelungsstellung verlagert, beispielsweise durch Verdrehen oder Verschieben. Dadurch werden die Sperrkörper oder Widerstandselemente von der Oberfläche des Liners weggebogen, eine Sperrschicht zwischen Liner und Prothesenschaft gelegt oder eine andere Entkopplung bewirkt, wodurch sich der Liner aus dem Prothesenschaft herausziehen lässt. Die Trenneinrichtung kann auch als Folie ausgebildet sein. Über eine entsprechende Trenneinrichtung ist es möglich, aus einem geschlossenwandigen Schaft problemlos auszusteigen, ohne größere Kräfte aufzuwenden.

Die Ausführungen zu dem beanspruchten Prothesenschaftsystem gelten dementsprechend auch für Prothesenschaftsysteme ohne zusätzliche Kopplungseinrichtung im distalen Abschlussbereich.

Der Prothesenschaft zur Verwendung in einem oben beschriebenen Prothesenschaftsystem weist einen distalen Abschlussbereich, zumindest eine sich davon in proximaler Richtung erstreckende Schaftwand und Anschlussmittel zur Befestigung einer Prothesenkomponente auf, wobei die Schaftwand einen in dem Prothesenschaft aufgenommenen Stumpf im angelegten Zustand zumindest teilweise umschließt und in dem distalen Abschlussbereich eine Magnetkopplung angeordnet ist, und sieht vor, dass an der Innenseite der Schaftwand zumindest ein Widerstandsbereich angeordnet oder ausgebildet ist, der entgegen einer Auszugsbewegung eines Liner in Proximalrichtung aus dem Prothesenschaft wirkt und dass der zumindest eine Widerstandsbereich mit gleichgerichteten knickstabilen Noppen, Stegen oder Fasern ausgestattet ist.

In einer Weiterbildung der Erfindung ist vorgesehen, dass der Prothesenschaft radial aufweitbar ist, sodass das Ablegen des Prothesenschaftes erleichtert wird. Die Kopplung zwischen den Widerstandsbereichen wird aufgehoben oder zumindest signifikant verringert, so dass durch das Verlagern des Prothesenschaftes von dem Liner weg eine Auszugsbewegung erleichtert wird.

Der Prothesenschaft kann mehrere zueinander verschwenkbare oder klappbare Schaftwandkomponenten aufweisen, wobei an dem Prothesenschaft zumindest eine Spanneinrichtung angeordnet sein kann, die eine Umfangsverringerung der Schaftwand bewirkt. Der Prothesenschaft kann in oder entgegen einer radialen Aufweitbewegung elastisch vorgespannt sein und gegebenenfalls mit einem Gurtsystem ausgebildet sein, wodurch der Prothesenschaft selbstschließend ausgebildet ist. Bei einer Vorspannung entgegen einer Aufweitbewegung ist der Prothesenschaft über die elastische Vorspannung selbstschließend ausgebildet.

Der Prothesenschaft ist vorzugsweise modular ausgebildet und weist einen Träger und daran befestigte, separat gefertigte Schaftwandkomponenten auf, um eine Individualisierung industriell vorgefertigter Komponenten einfach vornehmen zu können. Die Schaftwandkomponenten können reversibel an dem Träger oder an daran angeordneten oder befestigten Befestigungselementen angeordnet sein, um eine Anpassung an sich verändernden Bedingungen an dem Patienten zu ermöglichen.

In dem distalen Abschlussbereich des Prothesenschaftes und/oder in dem Linerendbereich können Magnete, insbesondere Permanentmagnete mit alternierender Polung oder ein ferromagnetisches Element angeordnet sein. Das ferromagnetische Element kann gleichzeitig als Widerlager für die Festlegung der distalen Anschlussmittel dienen und eine Befestigungseinrichtung, z.B. ein Gewinde, zu deren Fixierung aufweisen.

Der Widerstandsbereich auf der Innenseite der Seitenwand kann mit Formschlusselementen und/oder Adhäsionsbereichen und/oder Saugnäpfen oder einem Saugnapfbereich ausgebildet sein, um eine formschlüssige oder kraftschlüssige Kopplung mit der Außenseite eines Liners zu ermöglichen. Die Widerstandsbereiche können Magnete oder auch Klettverschlüsse aufweisen. Der Widerstandsbereich ist bevorzugt schaltbar ausgebildet oder weist schaltbare Widerstandselemente auf, so dass eingestellt werden kann, wann und in welchem Maß ein Widerstand gegen eine Auszugsbewegung bereitgestellt wird oder eine Auszugsbewegung blockiert oder freigegeben wird.

Ein Liner zur Verwendung in einem Prothesenschaftsystem, wie es oben beschrieben wurde, sieht eine proximale Einstiegsöffnung und eine sich in distaler Richtung erstreckende Seitenwand vor, die einen Stumpf im angelegten Zustand zumindest teilweise umgibt und im angelegten Zustand zwischen dem Stumpf und einem Prothesenschaft angeordnet ist, wobei in einem distalen Linerendbereich eine Magnetkopplung angeordnet ist, wobei an der Außenseite der Seitenwand des Liners zumindest ein Widerstandsbereich angeordnet und ausgebildet ist, der eine Auszugsbewegung des Liners in proximaler Richtung aus dem Prothesenschaft entgegen wirkt und der zumindest eine Widerstandsbereich mit Noppen, Stegen oder gerichteten Fasern ausgestattet ist.

Der Widerstandsbereich oder die Widerstandsbereiche können mit Formschlusselementen und/oder Adhäsionsbereichen ausgebildet oder ausgestattet sein. In dem distalen Linerendbereich können Magnete, insbesondere in alternierender Polung oder zumindest ein ferromagnetisches Element angeordnet sein, um eine Magnetkopplung mit einem Prothesenschaft zu bewirken. Die Magnete sind bevorzugt als Permanentmagnete ausgebildet.

Nachfolgend werden Ausführungsbeispiele der Erfindung anhand der beigefügten Figuren näher erläutert. Es zeigen:
- Figur 1-: die Explosionsdarstellung eines Prothesenschaftes;
- Figur 2-: einen Prothesenschaft im endmontierten Zustand
- Figur 3 -: eine Seitenansicht eines endmontierten Prothesenschaftes;
- Figur 4 -: eine Schrägdraufsicht gemäß Figur 3;
- Figur 5 -: eine Variante der Figur 1;
- Figur 6 -: eine Detailansicht eines Widerstandsbereiches;
- Figur 7 -: eine Schnittdarstellung durch einen Widerstandsbereich;
- Figuren 8 und 9 -: Ansichten von Prothesenlinern;
- Figur 10 -: eine Detailansicht eines Widerstandsbereiches mit einer Trenneinrichtung; sowie
- Figur 11 -: eine schematische Schnittdarstellung durch ein angelegtes Prothesenschaftsystem.

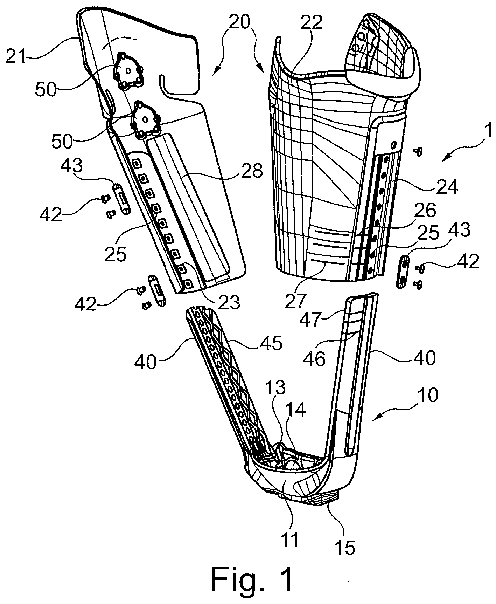

Figur 1 zeigt in einer Explosionsdarstellung einen Prothesenschaft 1 mit einem Träger 10, an dem zwei Schaftwandkomponenten 21, 22 lösbar befestigt sind. Im montierten und angelegten Zustand umgeben die beiden Schaftwandkomponenten 21, 22 den nicht dargestellten Stumpf vollumfänglich und bilden eine den Stumpf umschließende Schaftwand 20. In einer alternativen Ausführungsform kann die Schaftwand 20 auch einteilig ausgebildet sein und entweder einen geschlossenen Querschnitt oder einen offenen Querschnitt aufweisen.

Der Träger 10 in dem dargestellten Ausführungsbeispiel weist an seinem distalen Ende eine formstabile Kappe 11 auf, dessen distaler Seite nicht dargestellte Anschlussmittel für eine weitere Prothesenkomponente, beispielsweise ein Prothesenkniegelenk festlegbar sind. Diese formstabile Kappe 11 dient als Auflage für das nicht dargestellte Stumpfende und weist zumindest eine Halteeinrichtung 14 für eine Festlegung oder Sicherung des Stumpfes oder an einem Stumpf angelegten Liners an dem Prothesenschaft 1 auf. Diese Halteeinrichtung 14 ist im dargestellten Ausführungsbeispiel als Klettverschlusskomponente ausgebildet, in einer alternativen Ausführungsform können andere Formschlusseinrichtungen oder auch eine Magnetsicherung eingebaut sein, um über korrespondierende Formschlusselemente oder ferromagnetische Komponenten eine Sicherung des distalen Endes des Liners an dem Träger 10 und damit an dem Prothesenschaft zu bewirken.

Von der formstabilen Kappe 10 erstrecken sich in Proximalrichtung zwei Befestigungselemente 40, die als Schienen ausgebildet sind. Die Befestigungselemente 40 sind im angelegten Zustand des Prothesenschaftes 1 auf der medialen und lateralen Seite des Stumpfes positioniert und können entweder eine gleiche Länge oder aber unterschiedliche Längen aufweisen, wobei auf der lateralen Seite bevorzugt eine größere Länge vorgesehen ist. Innerhalb der als Schienen ausgebildeten Befestigungselemente 40 sind Durchgangsbohrungen 45 als Formschlusselemente zur Festlegung der Schaftwandkomponenten 21, 22 angeordnet. Die Durchgangsbohrungen 45 können mit einem Gewinde versehen sein und sind in einem definierten Abstand zueinander hintereinander in Längserstreckung der Befestigungselemente 40 angeordnet. Das lateral angeordnete Befestigungselement 40 ist über ein Scharnier 13 klappbar an dem Träger 10 angeordnet, das mediale Befestigungselement 40 ist einstückig an der distalen Kappe 11 ausgebildet, so dass sich ein L-förmiger, starrer Grundkörper ausbildet. Neben einer schwenkbaren Lagerung des medialen Befestigungselementes 40 kann dieses auch verschieblich an dem Träger 10 angeordnet sein, um eine Anpassbarkeit der Stumpfweite im distalen Bereich zu ermöglichen. Grundsätzlich ist es auch möglich, beide Befestigungselemente 40 klappbar auszugestalten oder aber auch das mediale Befestigungselement 40 starr an der formstabilen Kappe 11 anzuordnen oder damit auszubilden. Auch sind mehr als zwei Befestigungselemente 40 an dem Träger ausbildbar oder können dort angeordnet werden.

Die Schaftwandkomponenten 21, 22 und damit die gesamte Schaftwand 20 sind lösbar an dem Träger 10 befestigt. Dazu sind an den Außenseiten der Schaftwandkomponenten 21, 22 Führungen 23, 24 für die Befestigungselemente 40 befestigt. Die Führungen 23, 24 sind als Schienenaufnahmen ausgebildet, die korrespondierend zu den schienenartigen Ausgestaltungen der Befestigungselemente 40 ausgebildet sind und diese in sich aufnehmen können. Die Führungen 23, 24 können C-förmig ausgebildet sein, wobei der Querschnitt der Führungen 23, 24 entweder ein seitliches Einführen der Befestigungselemente 40 zulässt oder aber aufeinander zu gerichtete Dachschenkel aufweist, so dass die jeweiligen Befestigungselemente 40 nur in Längserstreckung in die entsprechende Führung 23, 24 eingeschoben werden kann. Die Seitenwände der Führung 23, 24 kann auch zueinander geneigt ausgebildet sein, so dass die Befestigungselemente 40 sowohl in Medialrichtung als auch in Lateralrichtung fixiert jedoch weiterhin in Längserstreckung der Befestigungselemente 40 verschieblich in den Führungen 23, 24 geführt sind.

In den Führungen 23, 24 sind korrespondierend zu den Durchgangsbohrungen 45 in den Befestigungselementen 40 Bohrungen mit Innengewinden 25 angeordnet, die in Abständen zueinander in Längserstreckung hintereinander angeordnet sind, in denen sie mit den Durchgangsbohrungen 45 fluchten. Über Formschlusselemente 42 in Gestalt von Schrauben werden die Schaftwandkomponenten 21, 22 mit dem jeweiligen Befestigungselement 40 verbunden. Dazu werden die Befestigungselemente 40 in die Führungen 23, 24 eingeführt, bis die gewünschte Länge erreicht ist. Die Durchgangsbohrungen 45 und die Innengewinde 25 innerhalb der Führungen 23, 24 werden zueinander ausgerichtet und die Schrauben 42 hindurchgesteckt und fixiert. Dadurch wird eine formschlüssige, lösbare Festlegung der Schaftwand 20 an dem Träger 10 erreicht. Die Durchgangsbohrungen 45 können auch als Langlöcher ausgebildet sein, um eine quasi stufenlose Einstellung der Position der Schaftwandkomponenten 21, 22 relativ zu dem Träger 10 zu ermöglichen. Es kann auch ein sich über die Gesamtlänge erstreckendes Langloch ausgebildet sein, so dass neben einer formschlüssigen Fixierung der Schaftwand 20 an den Befestigungselementen 40 die Längsverschieblichkeit entlang der Führungen 23, 24 klemmend über die Schrauben 42 und eine Unterlegscheibe 43 blockiert wird.

Im distalen Bereich der Schaftwandkomponenten 21, 22 sind Markierungen 26 und Materialschwächungen 27 quer zur Längserstreckung ausgerichtet angebracht, um bei Notwendigkeit die Gesamtlänge kürzen zu können. Die Materialschwächungen 27 können sich über den gesamten Umfang der Schaftwandkomponente 21, 22 erstrecken, bei einer einteiligen Ausgestaltung der Schaftwand 20 vollumfänglich. Die Markierungen 26 sind bevorzugt in gleichmäßigen Abständen zueinander orientiert und können auch mit einer Skala versehen sein, um eine Längenanpassung zu erleichtern. Korrespondierende Markierungen 46 und Materialschwächungen 47 sind an den Befestigungselementen 40, vorzugsweise an deren proximalen Enden angeordnet und ermöglichen eine individuelle Anpassbarkeit der Länge des gesamtes Prothesenschaftes 1.

Die Schaftwandkomponenten 20, 21 bilden als Schaftwand 20 einen proximalen Schaftanteil, der Träger 10 mit den Befestigungselementen 40 bildet einen distalen Schaftanteil, die längsverschieblich zueinander und aneinander festlegbar ausgebildet und angeordnet sind, so dass sie entweder stufenlos oder stufenverstellbar und fixierbar zueinander sind. Durch ein Kürzen des distalen Schaftanteils in Gestalt des Trägers 10 und/oder des proximalen Schaftanteils in Gestalt der Schaftwand 20 kann eine Verringerung der Schaftlänge erzielt werden, wodurch es möglich ist, industriell vorgefertigte Schaftteile einzusetzen, um einen vorgefertigten Schaft an individuelle Stumpflängen anpassen zu können. Auch kann die gewünschte Belastung auf das Stumpfende angepasst und eingestellt werden.

An der lateralen Schaftwandkomponente 21 ist eine Spanneinrichtung 50 angeordnet, über die beispielsweise über ein Schnur-Kabel-Gurtsystem die Schaftweite variiert werden kann. Aufgrund der zweiteiligen Ausgestaltung der Schaftwand 20 im dargestellten Ausführungsbeispiel sowohl der klappbaren Lagerung der lateralen Schaftwandkomponente 21 an dem Träger 10 ist es möglich und notwendig, die Position der Schaftwandkomponenten 21, 22 zueinander in Umfangsrichtung zu fixieren. Über die Klappbarkeit des Trägers 10 und die dadurch erreichbare Aufweitbarkeit des Prothesenschaftes 1 ist es möglich, dass Einsteigen in den Prothesenschaft zu erleichtern. Ebenfalls ist es dadurch möglich, Formschlusselemente 28 an der Innenseite der Schaftwand anzuordnen oder auszubilden, die eine Bewegung in Proximalrichtung behindern oder verhindern. So kann an der Innenseite der Schaftwand 20 ein Klettverschluss, ein Steigfell oder eine Oberflächenstrukturierung angeordnet oder ausgebildet sein, die formschlüssig in eine entsprechende Struktur an dem Liner eingreift. Durch die Aufklappbewegung kann ein Abschälvorgang oder außer Eingriff Bringen erfolgen, so dass eine entsprechende Trennung von Prothesenschaft 1 und Liner leicht möglich ist.

In dem dargestellten Ausführungsbeispiel der Figur 1 sind die Schaftwandkomponenten 21, 22 unterschiedlich ausgestaltet. Die mediale Schaftwandkomponente 22 erstreckt sich über mehr als die Hälfte des Gesamtumfanges, hier 3/4 des Gesamtumfanges, während die laterale Schaftwandkomponente 21 so bemessen ist, dass eine Umfangsergänzung vorliegt, so dass der Prothesenstumpf vollumfänglich umschlossen werden kann. Die Seitenkanten der Schaftwandkomponenten 21, 22 können im angelegten und zueinander festgelegten Zustand einander überlappen und ermöglichen dadurch eine Weiteneinstellbarkeit auch im Verlauf des Tragens, wenn Stumpfvolumenschwankungen vorliegen.

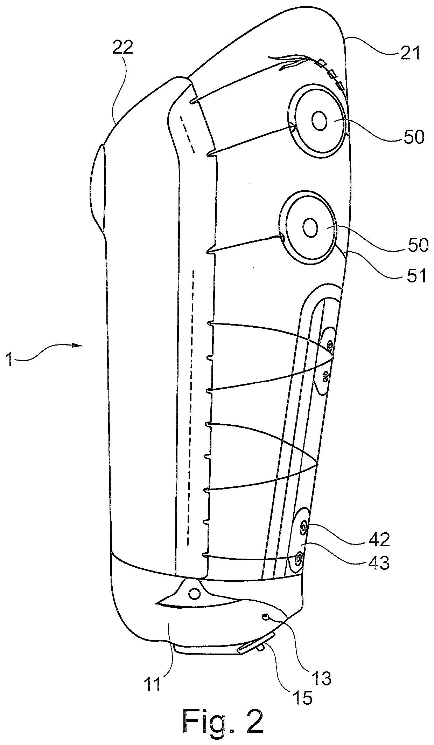

Ein fertig montierter Prothesenschaft 1 ist in der Figur 2 dargestellt. Es ist die distale, formstabile Kappe 11, die Scharniereinrichtung 13 für das laterale Befestigungselement 40 sowie die Schaftwand 20 aus einer lateralen Schaftwandkomponente 21 und einer medialen Schaftwandkomponenten 22 zu erkennen. Die Spanneinrichtung 50 weist im dargestellten Ausführungsbeispiel zwei Drehmechanismen auf, über die zwei Kabel 51 in ihrer effektiven Länge veränderbar sind. Die Kabel oder Bänder 51 sind an dem einander gegenüberliegenden Seitenrändern der medialen Schaftwandkomponente 22 angeordnet und bewirken durch eine Verkürzung eine Umfangsverringerung. Durch Drehen der Spanneinrichtungen 50 in entgegengesetzte Richtung kann der Prothesenschaftumfang erweitert werden. Die laterale Schaftwandkomponente 21 ist an dem Träger 10 über insgesamt vier Schrauben 42 lösbar fixiert. Die Schrauben 42 sind durch die Durchgangsbohrungen 45 in dem Befestigungselement 40 hindurch geführt und in die Innengewinde 25 innerhalb der Führung 23 eingeschraubt. Durch die Unterlegscheibe 43 ergibt sich eine zusätzliche Klemmwirkung.

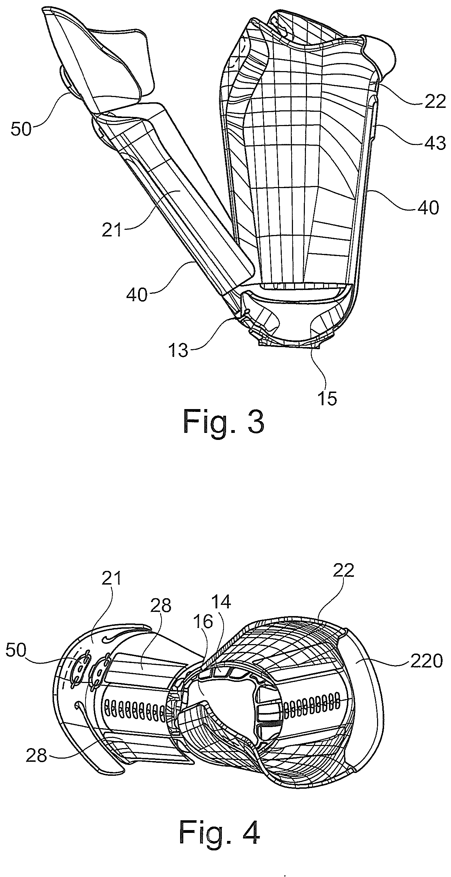

Figur 3 zeigt in einer Seitenansicht den Prothesenschaft 1 in einem aufgeklappten Zustand, wobei die beiden Schaftwandkomponenten 21, 22 bereits an dem Träger 10 befestigt sind. Die beiden Schaftwandkomponenten 21, 22 erstrecken sich bis zu der formstabilen Kappe 11 im montierten Zustand, so dass der Prothesenschaft 1 den nicht dargestellten Stumpf mit dem Liner nahezu vollständig umschließt. Der Figur 3 ist ebenfalls zu entnehmen, dass die linke Schaftwandkomponente 21 die rechte Schaftwandkomponente 22 teilweise an den Rändern überdeckt, so dass der Prothesenschaft 1 über nahezu die gesamte Länge den Stumpf vollständig umgibt.

**In** der Figur 4 ist der Prothesenschaft 1 in der Ausgestaltung gemäß Figur 3 in einer Schrägdraufsicht gezeigt. An der frontalen Schaftwandkomponente 21 sind die Spanneinrichtungen 50 zu erkennen, ebenso sind die Widerstandsbereiche 28 an der Innenseite der frontalen Schaftwandkomponente 22 zu erkennen. Die zweite Schaftwandkomponente 22 umgibt den nicht dargestellten Stumpf um mehr als Dreiviertel des Umfanges. Aufgrund des offenen Querschnittes der Schaftwandkomponente 22 ist ein Aufweiten des Prothesenschaftes 2 in Umfangsrichtung möglich. An dem proximalen, posterioren Randbereich der posterioren Schaftwandkomponente 22 ist eine Polsterung 220 angeordnet, die aus einem weicheren Material als die übrige Schaftwandkomponente 22 besteht, um eine Abpolsterung bereitzustellen, durch die der Tragekomfort erhöht wird. An der Innenseite der zweiten Schaftwandkomponente 22 sind ebenfalls Widerstandsbereiche 28 ausgebildet, die mit dem nicht dargestellten Prothesenliner in Wechselwirkung treten und einen Widerstand gegen eine Auszugsbewegung aus dem Prothesenschaft 2 bereitstellen oder eine formschlüssige Verriegelung zwischen dem Liner und dem Prothesenschaft 1 ausbilden.

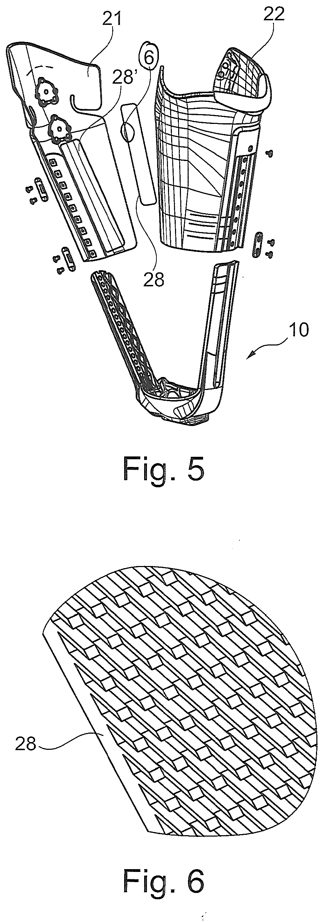

Figur 5 zeigt eine Variante der Figur 1 mit dem Prothesenschaft 1 in einem noch nicht montierten Zustand. Statt der in der frontalen Schaftwandkomponente 22 ausgebildeten Widerstandsbereiche 28, die beispielsweise durch eine entsprechende Riffelung, eine sägezahnartige Ausgestaltung der Oberfläche, durch Noppen, Rillen oder eine andere Profilierung bereitgestellt werden, ist an der frontalen Schaftwandkomponente 21 ein Befestigungsbereich 28'für einen separat gefertigten Widerstandsbereich 28 angeordnet. Der Widerstandsbereich 28 oder die Widerstandsbereiche 28 sind lösbar an der jeweiligen Schaftwandkomponente 21, 22 befestigt und ermöglichen so einen Austausch bei einem Verschleiß oder eine Anpassung an den jeweiligen Nutzer, beispielsweise um eine erhöhte Haftung des Liners an dem Prothesenschaft 1 zu ermöglichen.

Die Oberfläche des Widerstandsbereiches 28 ist vergrößert in der Figur 6 dargestellt. Es sind gleichgerichtete Stege, Noppen oder Fasern zu erkennen, die in Richtung auf das distale Ende oder die formstabile Kappe 11 geneigt sind, so dass bei einem Hineingleiten in den Prothesenschaft 1 kein oder nur ein geringer Widerstand der Einführbewegung entgegengesetzt wird. Durch das Hineingleiten legen sich die Noppen, Stege oder Fasern an die Schaftinnenwand an und ermöglichen ein Entlanggleiten des Prothesenliners beim Einführen. Bei einer entgegengesetzt gerichteten Bewegung verhaken sich die Spitzen der Stege, Noppen oder Fasern und richten sich auf, wodurch ein radialer Druck auf den Prothesenliner ausgeübt und gleichzeitig eine Auszugsbewegung aufgrund der durch die Stege, Noppen oder Fasern bereitgestellten Widerstände blockiert wird. Die Stege, Noppen oder Fasern können vergleichsweise knickstabil ausgebildet sein, so dass ein Umbiegen bei einem Ausziehen oder einer Bewegung entgegen der Einführrichtung nicht oder nur sehr schwer möglich ist.

Zum Ablegen des Prothesenschaftes 1 kann die Spanneinrichtung 50 geöffnet und die eine Schaftwandkomponente 21 von dem Prothesenliner abgeklappt und/oder der offene Querschnitt der anderen Schaftwandkomponente 22 aufgebogen werden, so dass der Liner mit dem Stumpf leicht aus dem Prothesenschaft 1 entfernt werden kann. Es müssen lediglich die Magnetkräfte durch die Magnetkopplung im distalen Ende überwunden werden. Die dazu an dem Prothesenliner 2 angeordneten Magnete können an der Metallplatte 16, die auf der Innenseite der formstabilen Kappe 11 vorhanden ist, anhaften.

Eine Variante zum Lösen oder zum Bereitstellen einer Ausstiegsmöglichkeit aus dem Prothesenschaft 1 ist dadurch gegeben, dass ein Trennschild zwischen die Außenoberfläche des Prothesenliners und die Innenoberfläche des Prothesenschaftes 1 eingeführt wird, beispielsweise ein Kunststoffschieber, der flach ausgebildet ist und sich an die Kontur des Stumpfes anschmiegt. Sobald die formschlüssig wirkenden Widerstandseinrichtungen, wie Stege, Noppen, Strukturelemente oder gerichtete Fasern der Widerstandsbereiche 28 durch den Schieber oder die Trenneinrichtung abgedeckt sind, findet keine Wechselwirkung mehr zwischen den Widerstandsbereichen statt und der Prothesenliner kann zusammen mit dem Stumpf einfach aus dem Prothesenschaft 1 herausgezogen werden.

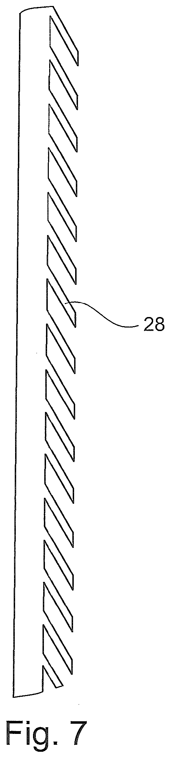

**In** der Figur 7 ist die Orientierung der Stege der Widerstandsbereiche 28 in einer Schnittdarstellung gezeigt. Es ist zu erkennen, dass eine glatte Außenwand vorhanden ist, auf deren gegenüberliegender Seite die in durch die Pfeilrichtung angedeutete Einführrichtung geneigten Strukturelemente, wie Stege, Rippen, Noppen oder dergleichen, angeordnet sind.

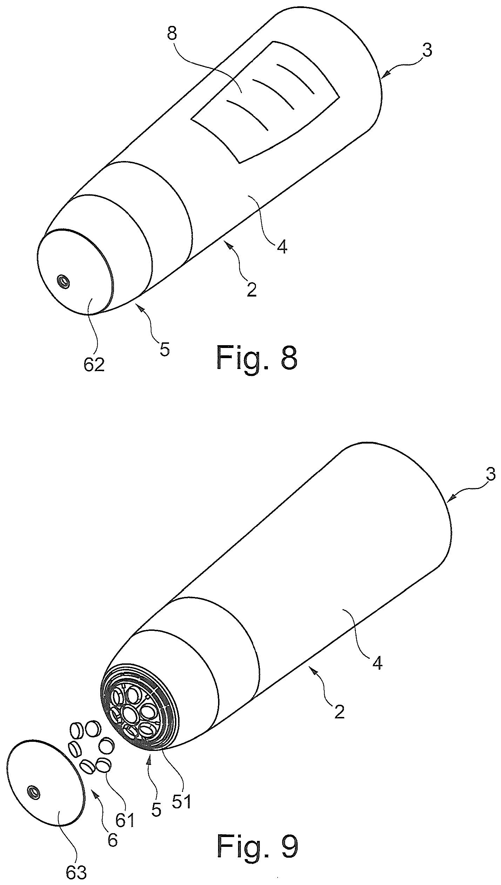

Figur 8 zeigt einen Prothesenliner 2 mit einer proximalen Einstiegsöffnung 3 und einer distalen Abschlusskappe in einem distalen Linerendbereich 5. Die distale Abschlusskappe kann beispielsweise als eine ferromagnetische Platte 62 ausgebildet sein, die ein Gewinde zur Aufnahme eines Verriegelungselementes, beispielsweise eines Pins, oder für einen Anschluss für eine Saugeinrichtung aufweist. Von dem distalen Linerendbereich 5 erstreckt sich in Proximalrichtung eine geschlossene Seitenwand 4, an deren Außenseite ein Widerstandsbereich 8 angeordnet ist, der sich im dargestellten Ausführungsbeispiel bis kurz unterhalb der proximalen Einstiegsöffnung 3 erstreckt. Der Widerstandsbereich 8 kann durch eine Formgebung einen formschlüssigen Eingriff mit den Komponenten der korrespondierend ausgebildeten Widerstandsbereiche 28 der Schaftinnenseite ausbilden. Zusätzlich oder alternativ können Adhäsionsbereiche an der Außenseite der Seitenwand 4 des Prothesenliners 2 angeordnet oder ausgebildet sein, um einen erhöhten Wiederstand gegen eine Auszugsbewegung des Liners 2 in Proximalrichtung aus dem Prothesenschaft 1 bereitzustellen. Der Widerstandsbereich 8 kann auch als Gewebe, als Flausch, als Strichvelour, als gerichtete Fasern, Noppen, Stege, Rippen, Magnetbereich, Klettverschluss oder Saugnapfbereich oder mit Saugnäpfen oder dergleichen ausgebildet sein. Das Material des Liners 2 im Bereich der Seitenwand 4 ist bevorzugt elastisch. Vorteilhafter Weise besteht der Liner aus einem Elastomer wie Silikon, Polyurethan oder TPE. Alternativ kann der Liner auch aus einem Abstandsgewirk bestehen. Vorteilhafterweise ist in diesem Fall das Abstandsgewirk auf der körperzugewandten Seite zumindest teilweise mit einem haftenden Elastomer beschichtet, um ein Ausziehen zu verhindern. Ein Abstandsgewirk hat den Vorteil, da dieses atmungsaktiv sein kann. Der Liner selbst kann mit einem Textil oder einer reibungsminimierenden Oberflächenbeschichtung versehen sein, um das Einsteigen zu erleichtern. Das Textil kann als Widerstandsbereich dienen, so dass kein zusätzlicher Widerstandsbereich notwendig ist. Im oder an dem Liner kann zusätzlich ein Matrix vorhanden sein, die den sogenannten Melkeffekt verhindert, indem die Längselastizität reduziert wird.

Eine Variante des Prothesenliners 2 ist in der Figur 9 gezeigt, bei der unterhalb einer distalen Abschlusskappe 63 Magnete 61 angeordnet sind, die in Ausnehmungen innerhalb des distalen Linerendbereiches 5 angeordnet sind. Dazu sind Aufnahmen oder Halterungen für die Magnete 61 in einer Linerendkappe 51 vorgesehen. Die Magnete 61 können eine alternierende Polung aufweisen, so dass eine Winkelorientierung und Ausrichtung des Liners 2 relativ zu beispielsweise korrespondierend angeordneten Magneten in der formstabilen Kappe 11 möglich sind. Neben einer Haftung über den magnetischen Schluss im Linerendbereich 5 sowie die Haftung, Verriegelung oder den Widerstand durch die Widerstandsbereiche 8, 28 wird dadurch eine Verdrehsicherung des Prothesenschaftes 1 relativ zu dem Liner 2 bewirkt. Dadurch ergibt sich eine erhöhte Sicherheit für den Nutzer des Prothesenschaftsystems aus dem Prothesenschaft 1 in Verbindung mit dem dargestellten Prothesenliner 2.

Über ein ferromagnetisches Element 16, 62 in Verbindung mit Permanentmagneten 14, 61 oder aber durch eine unmittelbare magnetische Kopplung mehrerer Permanentmagnete miteinander ist es möglich, zusätzlich zu einer haftenden oder formschlüssigen Verbindung in dem Seitenwandbereich und dem Prothesenschaftbereich eine Verriegelung im distalen Endbereich bereitzustellen, die eine präzise Ausrichtung des Liners 2 mit dem Stumpf innerhalb des Prothesenschaftes 1 ermöglicht, ohne jedoch ein hochgenaues Einführen einer formschlüssigen Verriegelung beispielsweise in Gestalt eine Pinlocks zu benötigen.

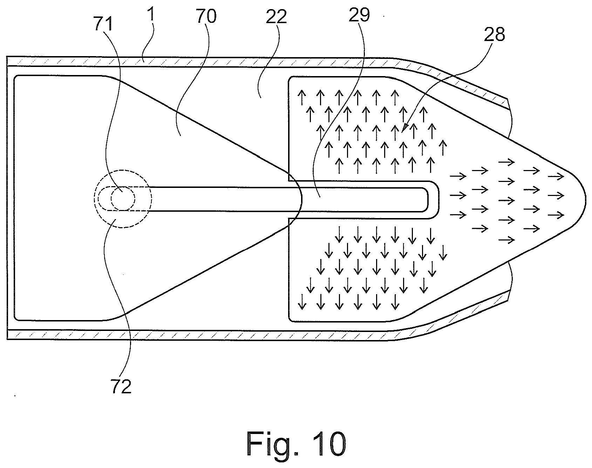

Figur 10 zeigt in einer schematischen Schnittdarstellung einen Prothesenschaft 1 mit auf der Innenseite angeordneten Sperrkörpern oder Widerstandselementen, die in einem Widerstandsbereich 28 angeordnet sind. Die Pfeile im Widerstandsbereich 28 deuten auf die unterschiedlichen Widerstandsrichtungen hin, in denen ein maximaler Widerstand gegen eine Bewegung entlang der Oberfläche bereitgestellt wird. Neben unterschiedlichen Widerstandsrichtungen ist es auch möglich, dass ein Widerstand nur entgegen einer Richtung, beispielsweise einer Einführrichtung des Prothesenschaftes, bereitgestellt wird.

Innerhalb des Prothesenschaftes 1 ist in der Schaftwand 22 ein Längsschlitz 29 ausgebildet, in dem ein Zapfen 71 verschieblich gelagert ist. Der Zapfen 71 weist auf seiner Außenseite ein Betätigungselement 72 oder einen Griff auf, über das der Zapfen 71 entlang der Ausnehmung 29 verschoben werden kann. Das Betätigungselement 72 ist von der Außenseite des Prothesenschaftes 1 aus zugänglich, insbesondere ist es auf der Außenseite der Schaftwand 20 angeordnet. Auf der dem Betätigungselement 72 gegenüberliegenden, innerhalb des Prothesenschaftes 1 an der Schaftwand 22 anliegenden Seite ist eine Trenneinrichtung 70 angeordnet, die als Schieber, Trennschild oder Folie ausgebildet ist. Im dargestellten Ausführungsbeispiel ist die Trenneinrichtung 70 im Wesentlichen dreieckig ausgebildet und weist mit einer Spitze in Richtung auf das nur angedeutete distale Ende des Prothesenschaftes 1.

In dem dargestellten Zustand befindet sich die Trenneinrichtung 70 im Wesentlichen außer Eingriff mit dem Widerstandsbereich 28 und den darin oder daran angeordneten Sperrkörpern, Widerstandselementen oder dergleichen. Zum Trennen eines formschlüssigen Eingriffes der Widerstandsbereiche 8, 28 miteinander oder auch zum Aufheben einer kraftschlüssigen Verbindung oder zum Schalten der Widerstandselemente oder Sperrkörper aus einer Eingriffsstellung in eine Freigabestellung, in der der nicht dargestellte Prothesenliner aus dem Prothesenschaft 1 heraus bewegt werden kann, wird die Trenneinrichtung 70 nach rechts verschoben und über den Widerstandsbereich 28 verlagert. Dadurch werden die Widerstandsbereiche 8 und 28 des Liners und des Prothesenschaftes außer Eingriff gebracht, entkoppelt oder hinsichtlich ihrer Adhäsionskräfte oder Widerstandskräfte verringert und außer Wechselwirkung gebracht, so dass der Patient aus dem Prothesenschaft 1 aussteigen kann. Die Innenseite der Trenneinrichtung 70 ist bevorzugt glattwandig ausgebildet. Die Trenneinrichtung 70 kann flexibel ausgebildet sein, um sich der Kontur des Schaftes und des Stumpfes anzupassen.

Durch die unterschiedlichen Orientierungen der Widerstandselemente, Sperrkörper oder Widerstandsabschnitte in dem Widerstandsbereich 28 ist neben einer Verringerung einer Neigung zur Auszugsbewegung auch eine Fixierung des nicht dargestellten Prothesenliners innerhalb des Prothesenschaftes 1 hinsichtlich einer Rotation um eine Längsachse gegeben. Die Rotationsstabilität eines Prothesenschaftes relativ zu einem Prothesenliner ist ein wesentlicher Faktor für das Stabilitätsempfinden eines Nutzers und kann durch die unterschiedlich gerichteten Orientierungen, beispielsweise durch unterschiedlich gerichtete Borsten, Fasern, Stege, Schuppen, Rinnen, Oberflächenstrukturen oder dergleichen, bereitgestellt werden. Die Widerstandselemente können elastisch gelagert oder flexibel ausgebildet sein.

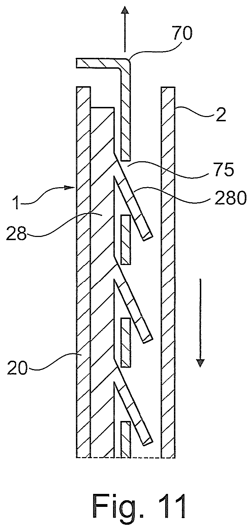

Eine weitere Variante der Trenneinrichtung ist in der Figur 11 dargestellt, in der eine schematische Teilansicht eines Prothesenschaftsystems mit einem Prothesenschaft 1 und einem darin angeordneten Liner 2 gezeigt ist. Auf der Innenseite der Schaftwand ist ein Widerstandsbereich 28 aufgebracht, der nach innen ragende Widerstandselemente 280 oder Sperrkörper aufweist und die in Richtung auf eine Einführrichtung, die durch den Pfeil nach unten angedeutet ist, geneigt sind. Eine Trenneinrichtung 70 ist mit Ausnehmungen 75 versehen, durch die die Widerstandselemente 280, die im dargestellten Ausführungsbeispiel als eine Vielzahl von Stegen, Borsten oder Rippen ausgebildet sind, hindurchragen. **In** der dargestellten Position befinden sich die Widerstandselemente 280 in Wechselwirkung mit der Außenseite des Prothesenliners 2 und ermöglichen aufgrund ihrer Neigung ein Einführen des Prothesenliners 2 in den Prothesenschaft 1. Eine entgegengesetzte Bewegung wird durch die Widerstandselemente 280 verhindert oder zumindest gehemmt, da sich die Widerstandselemente 280 oder Sperrkörper gegen die Außenwand des Prothesenliners 2 anlegen würden, nach oben gebogen werden würden und dadurch einen Widerstand gegen eine Auszugsbewegung bereitstellen, da sich die Widerstandselemente 280 verkeilen würden. Dieser Effekt kann durch ein Herausziehen oder nach oben Ziehen der als Schieber ausgebildeten Trenneinrichtung 70 verstärkt werden. Beim nach oben Ziehen der Trenneinrichtung 70 werden die Widerstandselemente 280 oder Sperrkörper aufgerichtet und gegen die Außenwand des Prothesenliners 2 gepresst.

Um die Auszugsbewegung zu ermöglichen oder zu erleichtern, wird die Trenneinrichtung 70 in die Einführrichtung des Prothesenliners 2 verlagert. Dadurch werden die durch die Ausnehmungen 75 hindurchgeführten Widerstandselemente 280 in ihrer Neigung verstärkt und gegen die Schaftwand 20 bzw. gegen die Oberfläche des Widerstandsbereiches 28 gedrückt und außer Eingriff mit dem Prothesenliner 2 gebracht. Die Spitzen oder Enden der Widerstandselemente 280 befinden sich dann innerhalb der Ausnehmungen 75 und werden durch die Innenoberfläche der Trenneinrichtung 70 überragt, so dass keine oder nur eine geringe Wechselwirkung zwischen den Widerstandselementen 280 und dem Prothesenliner 2 vorhanden ist. Der Prothesenliner 2 kann dann aus dem Prothesenschaft 1 herausgezogen werden. Zur Bereitstellung eines erhöhten Widerstandes wird die Trenneinrichtung 70 nach oben verlagert und die Widerstandselemente 280 aufgerichtet und in ihrer Neigung verändert.

## Patentansprüche

1. Prothesenschaft zur Verwendung in einem Prothesenschaftsystem mit einem Liner, mit einem distalen Abschlussbereich (11), zumindest einer sich davon in Proximalrichtung erstreckenden Schaftwand (20) und Anschlussmitteln (15) zur Befestigung einer Prothesenkomponente, wobei die Schaftwand (20) einen in dem Prothesenschaft (1) aufgenommenen Stumpf im angelegten Zustand zumindest teilweise umschließt und in dem distalen Abschlussbereich (11) eine Kopplungseinrichtung (14) angeordnet, wobei an der Innenseite der Schaftwand (20) zumindest ein Widerstandsbereich (28) angeordnet oder ausgebildet sind, der entgegen einer Auszugsbewegung eines Liners (2) in Proximalrichtung aus dem Prothesenschaft (1) wirkt, wobei der zumindest eine Widerstandsbereich (28) mit Noppen, Stegen oder gerichteten Fasern ausgestattet ist,
**dadurch gekennzeichnet, dass** die Noppen, Stege oder gerichtete Fasern knickstabil ausgebildet sind, so dass ein Umbiegen bei einem Ausziehen oder einer Bewegung entgegen der Einführrichtung nicht oder nur schwer möglich ist.

2. Prothesenschaft nach Anspruch 1, **dadurch gekennzeichnet, dass** der Prothesenschaft (1) radial aufweitbar ist.

3. Prothesenschaft nach Anspruch 1 oder 2, **dadurch gekennzeichnet, dass** der Prothesenschaft (1) mehrere, zueinander verschwenkbare oder klappbare Schaftwandkomponenten (21, 22) aufweist.

4. Prothesenschaft nach einem der voranstehenden Ansprüche, **dadurch gekennzeichnet, dass** an dem Prothesenschaft (1) zumindest eine Spanneinrichtung (50) angeordnet ist, die eine Umfangsverringerung der Schaftwand (20) bewirkt.

5. Prothesenschaft nach einem der voranstehenden Ansprüche, **dadurch gekennzeichnet, dass** der Prothesenschaft (1) in oder entgegen einer radialen Aufweitbewegung elastisch vorgespannt ist.

6. Prothesenschaft nach einem der voranstehenden Ansprüche, **dadurch gekennzeichnet, dass** der Prothesenschaft (1) selbstschließend ausgebildet ist.

7. Prothesenschaft nach einem der voranstehenden Ansprüche, **dadurch gekennzeichnet, dass** der Prothesenschaft (1) modular mit einem Träger (10) und daran befestigten, separat gefertigten Schaftwandkomponenten (21, 22) ausgebildet ist.

8. Prothesenschaft nach einem der voranstehenden Ansprüche, **dadurch gekennzeichnet, dass** in dem distalen Abschlussbereich (11) als Kopplungseinrichtung (14) Magnete, insbesondere in alternierender Polung, oder ein ferromagnetisches Element (16) angeordnet sind.

9. Prothesenschaft nach einem der voranstehenden Ansprüche, **dadurch gekennzeichnet, dass** der Widerstandsbereich (28) zusätzlich mit Formschlusselementen und/oder Adhäsionsbereichen ausgebildet ist.

10. Prothesenschaft nach einem der voranstehenden Ansprüche, **dadurch gekennzeichnet, dass** der Widerstandsbereich (28) schaltbar ausgebildet ist oder schaltbare Widerstandselemente aufweist.

11. Prothesenschaft nach einem der voranstehenden Ansprüche, **dadurch gekennzeichnet, dass** an dem Prothesenschaft eine Trenneinrichtung (70) verschieblich oder drehbar gelagert ist.

12. Liner zur Verwendung in einem Prothesenschaftsystem mit einem Prothesenschaft nach einem der voranstehenden Ansprüche, mit einer proximalen Einstiegsöffnung (3) und einer sich in distaler Richtung erstreckenden Seitenwand (4), die einen Stumpf im angelegten Zustand zumindest teilweise umgibt und im angelegten Zustand zwischen dem Stumpf und einem Prothesenschaft (1) angeordnet ist, wobei in einem distalen Linerendbereich (5) eine Kopplungseinrichtung (6) angeordnet, wobei an der Außenseite (5) der Seitenwand (4) des Liners (2) zumindest ein Widerstandsbereich (8) angeordnet oder ausgebildet ist, der einer Auszugsbewegung des Liners (2) in Proximalrichtung aus dem Prothesenschaft (1) entgegen wirkt, wobei der zumindest eine Widerstandsbereich (8) mit Noppen, Stegen oder gerichteten Fasern ausgestattet ist, **dadurch gekennzeichnet, dass** in dem distalen Linerendbereich (5) als Kopplungseinrichtung (6) Magnete (61) oder zumindest ein ferromagnetisches Element (62) angeordnet sind.

13. Liner nach Anspruch 12, **dadurch gekennzeichnet, dass** der Widerstandsbereich (8) zusätzlich mit Formschlusselementen und/oder Adhäsionsbereichen ausgebildet ist.

14. Liner nach Anspruch 12 oder 13, **dadurch gekennzeichnet, dass** die Magnete (61) in alternierender Polung angeordnet sind.

15. Prothesenschaftsystem mit einem Prothesenschaft (1) nach einem der Ansprüche 1 bis 11 und einem Liner (2) nach einem der Ansprüche 12 bis 14, wobei die Kopplungseinrichtungen (6, 14) in dem distalen Abschlussbereich (11) und dem distalen Linerendbereich (5) zwischen dem Liner (2) und dem Prothesenschaft (1) wirken und die Widerstandsbereiche (8, 28) miteinander in Wechselwirkung treten.

## Claims

1. A prosthetic socket for use in a prosthetic socket system with a liner, with a distal end region (11), at least one socket wall (20) extending therefrom in proximal direction and connection means (15) for securing a prosthetic component, wherein the socket wall (20), when in the applied state, at least partially encloses a stump that is received in the prosthetic socket (1), and a coupling device (14) arranged in the distal end region (11), wherein on the inner side of the socket wall (20), at least one resistance region (28) is arranged or formed which counteracts a movement extracting a liner (2) out of the prosthetic socket (1), in proximal direction , whereas the at least on resistance region (28) is provided with stays, naps or directed fibers, **characterized in that** the stays, naps or directed fibers are kink resistant, with the result that, when extracting or moving opposite the direction of introduction, bending is not possible, or possible only with difficulty.

2. The prosthetic socket according to claim 1, **characterized in that** the prosthetic socket (1) can be extended radially.

3. The prosthetic socket according to claim 1 or 2, **characterized in that** the prosthetic socket (1) has several socket wall components (21, 22) arranged swivelable or foldable to one another.

4. The prosthetic socket according to one of the preceding claims, **characterized in that**, at the prosthetic socket (1), at least one clamping device (50) is arranged which causes a reduction in circumference of the socket wall (20).

5. The prosthetic socket according to one of the preceding claims, **characterized in that** the prosthetic socket (1) is pretensioned elastically in or counter to a radial extension movement.

6. The prosthetic socket according to one of the preceding claims, **characterized in that** the prosthetic socket (1) is formed to be self-closing.

7. The prosthetic socket according to one of the preceding claims, **characterized in that** the prosthetic socket (1) is formed modularly with a support (10) and socket wall components (21, 22) secured thereto and produced separately.

8. The prosthetic socket according to one of the preceding claims, **characterized in that**, in the distal end region (11), magnets, in particular with alternating polarity, or a ferromagnetic element (16), are arranged as coupling device (14).

9. The prosthetic socket according to one of the preceding claims, **characterized in that** the resistance region (28) is additionally formed with form-locking elements and/or adhesion regions.

10. The prosthetic socket according to one of the preceding claims, **characterized in that** the resistance region (28) is formed switchable or has switchable resistance elements.

11. The prosthetic socket according to one of the preceding claims, **characterized in that** a separating device (70) is arranged at the prosthetic socket.

12. A liner for use in a prosthetic socket system according to one of the preceding claims, with a proximal entry opening (3) and a lateral wall (4) extending in distal direction, which, when in the applied state, at least partially surrounds a stump and, when in the applied state, is arranged between the stump and a prosthetic socket (1), wherein a coupling device (6) is arranged in a distal liner end region (5), wherein on the outer side (5) of the lateral wall (4) of the liner (2), at least one resistance region (8) is arranged or formed, which counteracts a movement extracting the liner (2) out of the prosthetic socket (1) in proximal direction, wherein the at least on resistance region (8) is provided with stays, naps or directed fibers, **characterized in that** in the distal liner end region (5) magnets (61) or at least one ferromagnetic element (62), are/is arranged as coupling device (6).

13. The liner according to claim 12, **characterized in that** the resistance region (8) is additionally formed with form-locking elements and/or adhesion regions.

14. The liner according to claim 12 or 13, **characterized in that** magnets (61) are arranged with alternating polarity.

15. Prosthetic socket system with a prosthetic socket (1) according to one of the claims 1 to 11 and a liner (2) according to one of the claims 12 to 14, wherein the coupling devices (6, 14) in the distal end region (11) and the distal liner end region (5) are acting between the liner (2) and the prosthetic socket (1) and that the resistance regions (8,28) interact with one another.

## Revendications

1. Emboîture de prothèse destinée à être utilisée dans un système d'emboîture de prothèse pourvu d'un manchon, comportant une zone de terminaison distale (11), au moins une paroi d'emboîture (20) s'étendant à partir de celle-ci en direction proximale, et des moyens de raccordement (15) destinés à fixer un composant de prothèse,
dans laquelle
à l'état mis en place, la paroi d'emboîture (20) entoure au moins partiellement un moignon logé dans l'emboîture de prothèse (1), et un dispositif d'accouplement (14) est disposé dans la zone de terminaison distale (11),
au moins une zone de résistance (28) est disposée ou formée sur la face intérieure de la paroi d'emboîture (20) et s'oppose à un mouvement d'extraction d'un manchon (2) dans la direction proximale hors de l'emboîture de prothèse (1),
ladite au moins une zone de résistance (28) est pourvue de bosses, de barrettes ou de fibres orientées,
**caractérisée en ce que** les bosses, barrettes ou fibres orientées sont conçues pour résister au flambage, de sorte qu'une déformation par flexion lors d'un retrait ou d'un mouvement en sens opposé à la direction d'insertion n'est pas possible ou n'est possible que difficilement.

2. Emboîture de prothèse selon la revendication 1,
**caractérisée en ce que** l'emboîture de prothèse (1) peut être évasée radialement.

3. Emboîture de prothèse selon la revendication 1 ou 2,
**caractérisée en ce que** l'emboîture de prothèse (1) comporte plusieurs composants de paroi d'emboîture (21, 22) pouvant pivoter ou se rabattre les uns par rapport aux autres.

4. Emboîture de prothèse selon l'une des revendications précédentes, **caractérisée en ce qu'**au moins un dispositif de serrage (50) est disposé sur l'emboîture de prothèse (1), lequel provoque une réduction de la circonférence de la paroi d'emboîture (20).

5. Emboîture de prothèse selon l'une des revendications précédentes, **caractérisée en ce que** l'emboîture de prothèse (1) est précontrainte élastiquement dans le sens d'un mouvement d'évasement radial ou en sens opposé à celui-ci.

6. Emboîture de prothèse selon l'une des revendications précédentes, **caractérisée en ce que** l'emboîture de prothèse (1) est conçue pour se fermer automatiquement.

7. Emboîture de prothèse selon l'une des revendications précédentes, **caractérisée en ce que** l'emboîture de prothèse (1) est conçue de manière modulaire avec un support (10) et des composants de paroi d'emboîture (21, 22) fabriqués séparément et fixés à celui-ci.

8. Emboîture de prothèse selon l'une des revendications précédentes, **caractérisée en ce que** des aimants, en particulier à polarité alternée, ou un élément ferromagnétique (16) sont disposés dans la zone de terminaison distale (11), en tant que dispositif d'accouplement (14).

9. Emboîture de prothèse selon l'une des revendications précédentes, **caractérisée en ce que** la zone de résistance (28) est en outre pourvue d'éléments de liaison par complémentarité de forme et/ou de zones d'adhérence.

10. Emboîture de prothèse selon l'une des revendications précédentes, **caractérisée en ce que** la zone de résistance (28) est conçue de manière commutable ou comporte des éléments de résistance commutables.

11. Emboîture de prothèse selon l'une des revendications précédentes, **caractérisée en ce qu'**un dispositif de séparation (70) est monté de façon mobile en translation ou en rotation sur l'emboîture de prothèse.

12. Manchon destiné à être utilisé dans un système d'emboîture de prothèse pourvu d'une emboîture de prothèse selon l'une des revendications précédentes, comportant une ouverture d'entrée proximale (3) et une paroi latérale (4) s'étendant dans la direction distale, laquelle, à l'état mis en place, entoure au moins partiellement un moignon et est disposée, à l'état mis en place, entre le moignon et une emboîture de prothèse (1),
dans lequel
un dispositif d'accouplement (6) est disposé dans une zone d'extrémité distale (5) du manchon,
au moins une zone de résistance (8) est disposée ou formée sur la face extérieure (5) de la paroi latérale (4) du manchon (2) et s'oppose à un mouvement d'extraction du manchon (2) dans la direction proximale hors de l'emboîture de prothèse (1),
ladite au moins une zone de résistance (28) est pourvue de bosses, de barrettes ou de fibres orientées,
**caractérisé en ce que** des aimants (61) ou au moins un élément ferromagnétique (62) sont disposés dans la zone d'extrémité distale (5) du manchon, en tant que dispositif d'accouplement (6).

13. Manchon selon la revendication 12,
**caractérisé en ce que** la zone de résistance (8) est en outre pourvue d'éléments de liaison par complémentarité de forme et/ou de zones d'adhérence.

14. Manchon selon la revendication 12 ou 13,
**caractérisé en ce que** les aimants (61) sont disposés en polarité alternée.

15. Système d'emboîture de prothèse comprenant une emboîture de prothèse (1) selon l'une des revendications 1 à 11 et un manchon (2) selon l'une des revendications 12 à 14,
dans lequel les dispositifs d'accouplement (6, 14) situés dans la zone de terminaison distale (11) et dans la zone d'extrémité distale (5) du manchon agissent entre le manchon (2) et l'emboîture de prothèse (1), et les zones de résistance (8, 28) interagissent entre elles.
